# EUROPEAN PATENT APPLICATION

(11) **EP 0 928 612 A2**
(43) Date of publication of application: **14.07.1999**
(21) Application number: 98123227.5
(22) Date of filing: 07.12.1998
(51) Int. Cl.: A61L 27/00

(54) **A method for treating a prosthesis having an apertured structure and associated devices**

(30) Priority: 10.12.1997 IT TO971068
(71) Applicant: SORIN BIOMEDICA CARDIO S.p.A., I-13040 Saluggia, Vercelli (IT)
(72) Inventor: Rinaldi, Stefano, 43100 Parma (Italy) (IT); Giannetti, Arnaldo, 13044 Crescentino (Vercelli) (IT); Pasquino, Enrico, 10149 Torino (IT)
(74) Representative: Bosotti, Luciano

(57) **Abstract**

The prosthesis, constituted entirely or in part from an apertured structure, is located in a chamber (11) which is taken to a level of sub-atmospheric pressure (27). Once the aforesaid level of sub-atmospheric pressure has been reached, a liquid (23), possibly containing pharmacological agents, is introduced into the chamber. Drawn by the sub-atmospheric pressure, the liquid saturates the apertured structure of the prosthesis. In this way, the risk is eliminated of air being trapped in the aforesaid apertured structure that could give rise to the formation of blood clots after implantation of the prosthesis. The aforesaid liquid can contain drugs that penetrate the prosthesis, performing their therapeutic action locally and over time after implantation.

## Description

The present invention generally concerns prostheses having an apertured structure. This term is here intended to indicate those prostheses (such as, for example, so-called vascular grafts or the suture rings of cardiac valve prostheses) constituted, entirely or in part, from tissue structures, spongy masses and/or having elaborate geometries with slits, cavities and spaces, that is apertures in general.

In use, these prostheses tend to hold air inside them with a consequent risk of the formation of blood clots in the period following implantation, due to the presence of air bubbles contained or otherwise held by the prosthesis.

The simple solution, sometimes adopted during the implantation operation, of immersing the prosthesis in, for example, a physiological saline bath, does not satisfactorily solve the problem, both because the results can depend, possibly significantly, on the time dedicated to this treatment and the ability of the person conducting it, and because it is in any case difficult to remove all the air from the prosthesis.

The present invention therefore aims to eliminate in radical manner the risk of the occurrence of these negative phenomena.

According to the present invention, this object is achieved by virtue of a method having the characteristics referred to in the following claims. The invention also concerns devices which can be used in the performance of this method.

An important advantage of the invention is that the liquid such as, for example, physiological saline that is introduced into the pores, even the deep pores, to replace the air that is naturally present, can be supplemented with drugs such as, for example, antibiotics, anti-thrombotic drugs, drugs that promote the integration between the prosthesis and the surrounding biological tissues, or growth factors. Because the liquid is held in the pores, the drug or drugs can exert their action locally and extended over time. In relation to this, it is also noted that the word "drug", as utilised in the present description and, where appropriate, in the accompanying claims, also includes the possible use of a binding agent applied to the prosthesis in order to attract thereto, with a binder-ligand association mechanism, pharmacologically active agents introduced into the patient's body.

The invention will now be described, purely by way of non-limitative example, with reference to the accompanying drawings, in which:
- Figure 1 shows a possible embodiment of a system for performing the method according to the invention;
- Figure 2 illustrates in greater detail the structure of one of the parts shown in Figure 1; and
- Figure 3 shows a container for a prosthesis suitable for use in the invention.

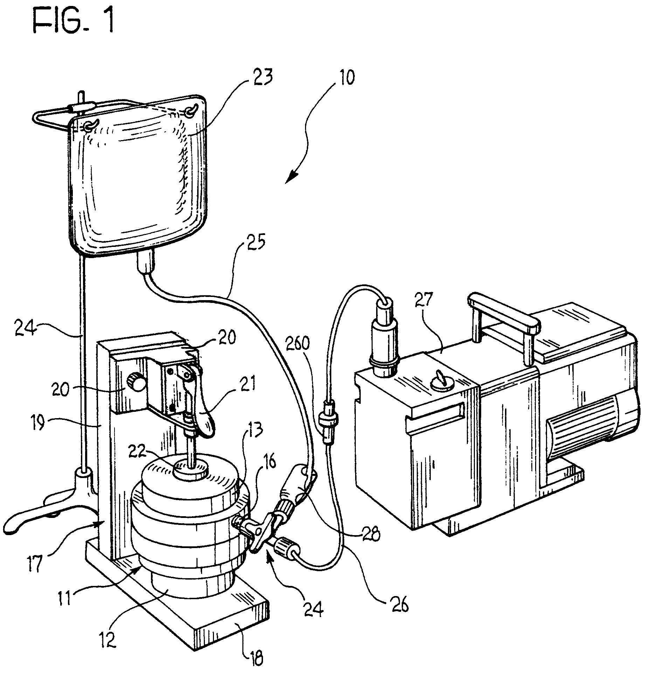

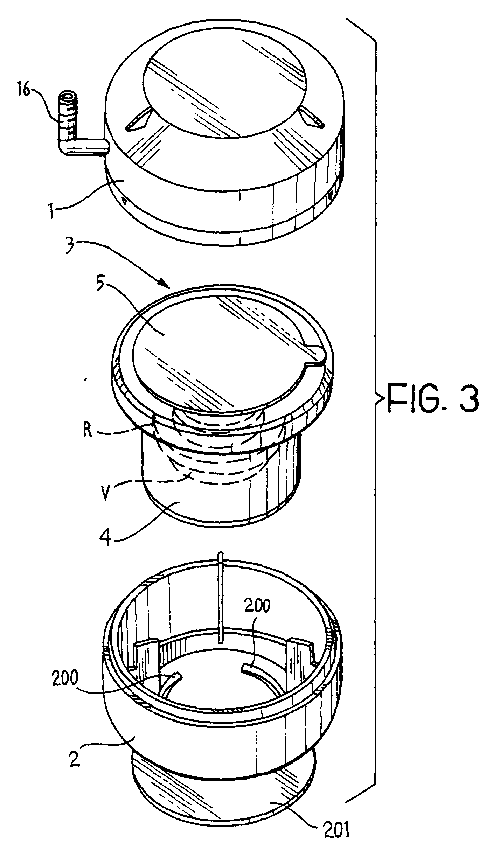

Before proceeding to the description of Figure 1, attention should be drawn to Figure 3. This drawing illustrates a container that can be used for the sterile packaging of a prosthesis such as a cardiac valve prosthesis.

With the exception of the presence of some characteristic elements (which will be referred to specifically below) relating to the application of the invention, the container illustrated in Figure 3 corresponds to the container currently utilised by the Assignee for cardiac valve prostheses sold by the Assignee itself under the commercial name Bicarbon™.

This container includes an outer shell formed from two cup-shape half shells 1 and 2 made from plastics material sealingly coupled (according to known criteria) along their respective mouth edges to define an inner chamber in which a further container 3 is located. This latter is constituted by a cup-like body 4 within which the valve prosthesis V is located. The body 4 is closed along its mouth part by a sealing disc 5. This latter is usually formed from a material able to form a sterile, although fluid-permeable, barrier. For example, it can be the material sold by the firm Du Pont under the commercial name Tyvek®.

With the exception of the innovative elements referred to above, which will be described better below, the container of Figure 3 is manufactured according to criteria that are widely known to the man skilled in the art. This makes the description of further details superfluous as they are not in themselves necessary in order to understand and put into effect the invention.

Of course, the above also applies to the valve prosthesis V. In this case, it is sufficient to note that the valve itself has a suture ring R of textile material, possibly with a spongy core, along its outer edge. In other words, it is a prosthesis having, at least in part, an "apertured" structure in the sense of the meaning attributed to this word in the introduction to the description.

The device illustrated in Figure 1, generally indicated 10, includes a vacuum bell 11 as its main element. The bell 11 is generally constituted from a casing that is sealably closed or closable so that it can be taken to a predetermined level of sub-atmospheric pressure (in short, to a "vacuum" state).

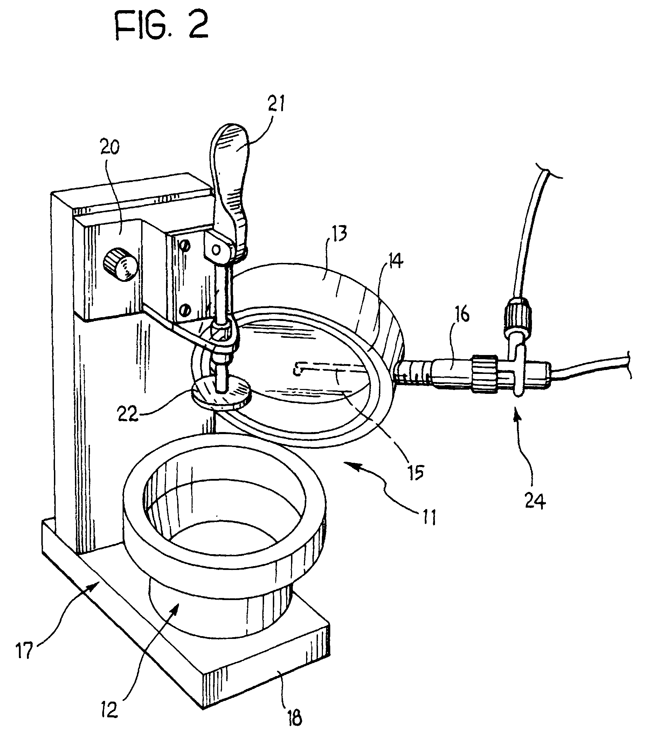

In the embodiment illustrated here (which is such that - as will be seen better below - the function of the bell 11 can also be performed, for example, by the prosthesis container), the bell 11 has the structure illustrated in greater detail in Figure 2, being formed from a cup-like holding body 12 with an associated closure cover 13.

The dimensions and shape of the cup-like body 12 are chosen so as to enable the prosthesis to be treated, possibly in its sterile container, to be introduced into the bell 11. In the embodiment illustrated here, the shape and dimensions of the bell 11 are such that it can accommodate the container 3 of the container shown in Figure 3.

Both the cup-like body 12 and the cover 13 can be formed, for example, from a material such as a metal material or, even better, at least as regards the cover 13, from a transparent plastics material such as polycarbonate or polymethacrylate so that it is possible to observe the inner chamber of the bell 11 from the outside during the treatment which will be described below.

The method according to the invention is preferably performed in the operating theatre, therefore in a sterile environment or close thereto. Consequently, the choice of materials forming the various parts of the device 10 must take account of this preference and be sterilisable.

The cover 13 has a sealing ring (a so-called O-ring) 14 along its outer edge, and is traversed by a radial duct 15 leading to one or more apertures intended to open into the upper part of the inner volume of the bell 11 when the cover 13 is fitted to the body 12. This duct 15 leads to its outer end with respect to the cover 13, and therefore to the bell 11, at a connector 16 (for example, of the type currently known as a "luer" connection) projecting out of the bell 11, for example, radially with respect thereto.

However, it is clear that the relative positioning of the parts described above is not in any way essential. For example, the sealing ring 14 could be located on the mouth part of the cup-like body 12, and the duct or ducts 15 could have different paths and/or be provided on the cup-like body 12, or partly on the cup-like body 12 and partly on the cover 13.

The reference numeral 17 generally indicates a clamp that enables the bell 11 to be positioned and the cover 13 to be held sealably closed on the mouth part of the cup-like body 12 during the treatment of the prosthesis.

In the embodiment illustrated here, the clamp 17 includes a base 18 defining a support surface for the bell 11, a pillar 19 extending vertically from the base 18, and an upper part 20 projecting over the bell 11 positioned on the base 18. The part 20 is substantially constituted by a toggle clamp element 21 of the type currently known as a Destaco clamp, leading to a pressure foot 22.

The method for positioning the bell 11 in the clamp 17 is clear from a comparison of Figure 2 in which the bell 11 is illustrated in an open position, and Figure 1 in which the bell 11, into which it is assumed that the container 3 containing the prosthesis V has already been introduced, has been positioned in the clamp 17; the toggle clamp 21 is then operated so that the foot 22 presses the cover 13 firmly against the mouth edge of the cup-like body 12.

The reference numeral 23 indicates a bag (or, generally, any other kind of container) located , for example, on an associated pillar or support 24, and containing a predetermined volume (for example, 200 cubic centimetres) of a liquid intended to be utilised for impregnating the apertured parts of the prosthesis V according to the methods that will be better illustrated below.

The liquid in question may be a so-called inert liquid such as physiological saline, or an active liquid constituted by, or rather containing, one or more drugs in the terms already referred to in the introduction to the description. These drugs can also be added to the inert liquid in the bag or container 23 through a suitable gate using a technique well known in medical practice.

The reference numeral 24 indicates a tap or valve (or any other equivalent fluid component) mounted on or otherwise connected to the connector 16 and which selectively connects this connector (and thus the duct 15)
- to a fluid line 25 that connects the tap 24 to the bag 23, or
- to a further fluid line 26 that connects the tap 24 to a source of sub-atmospheric pressure 27 (typically a vacuum pump or a vacuum line available wherever the treatment is effected, for example, in the operating theatre).

The lines 25 and 26 can be formed, for example, from flexible ducts made from soft polyvinylchloride with an internal diameter of, for example, approximately 1mm and an outer diameter of, for example, approximately 3.5mm.

Preferably, a so-called safety clamp 28, or any component able to interrupt the line 25 if necessary, is interposed in the line 25, usually close to the tap 24.

A filter 260 can be interposed at any point along the line 26 between the bell 11 and the source of sub-atmospheric pressure 27, which filter is able to constitute a sterile, fluid-permeable barrier.

In the tests conducted so far by the Applicant, a Leybold Trivac AF1.6 vacuum pump was used as the source of sub-atmospheric pressure.

With the possible application of the invention being directed towards the surgical field, this being preferable in many ways, the various parts of the device 10 and, in particular, the parts 12 and 13 of the bell 11, together with the associated accessories (the sealing gasket 14, the bag 23, the lines 25 and 26, the tap 24 etc.), are preferably formed from materials able to ensure the use of the method in a sterile environment.

This therefore means that components are preferably formed as single use components and/or components that are sterilisable using, for example, ethylene oxide.

Preferably, the system for generating and applying the sub-atmospheric pressure (the bell 11, the pump 27 and associated connections, in the embodiment illustrated here) are chosen and dimensioned so as to ensure that a typical level of sub-atmospheric pressure of approximately -850 mbar is reached in the inner chamber of the bell 11.

Usually, the system 10 is completed by a bath (not shown in the drawings) for collecting the liquid 23 that may be released on opening the bell 11.

Preferably, the assembly formed by the bell 11, the tap 24, the lines 25, 26 and, possibly, the bag 23 is in the form of a kit packaged in a sterile envelope.

This arrangement enables it to be used directly in the operating theatre, and therefore at the time of implanting a prosthesis, such as the valve prosthesis V, in the sequence of operations described below:
- removing the equipment described above and connecting it via the line 26 to the source of sub-atmospheric pressure 27,
- opening the outer container of the prosthesis by unrolling the adhesive strip (not shown) that connects the mouth parts of the half-shells 2 (it is recalled that the present description supposes that the prosthesis container corresponds to that illustrated in Figure 3 without the further elements that will be described below),
- positioning the inner container 3, in which the prosthesis V is located, in the cup-like body 12 of the bell 11,
- closing the bell 11 with the cover 13 and positioning the bell 11 in the clamp 17 which is then locked by acting on the closure device 21,
- starting the vacuum pump 27 (or, in any case, activating the line 26 as the source of sub-atmospheric pressure),
- on reaching the required level of sub-atmospheric pressure (in the case of the level of -850 mbar and the kind of pump 27 referred to above, this result can be achieved quickly, typically, in less than approximately 60 seconds), switching the tap 24 to the position that transfers the connector 16, and thus the duct 15, previously connected to the line 26, to the line 25, before opening the safety element 28, if present; at this point, the liquid in the bag 23 flows into the inner chamber of the bell 11, penetrating the inside of the container 3 (across the seal 5 which is, as said, permeable): in this way, the liquid, possibly containing one or more drugs, completely saturates the ring R - that is, the apertured part of the prosthesis - definitively preventing any air bubbles from being held therein or being able to enter it; if the liquid contains drugs, these become trapped in the pores of the apertured part of the prosthesis and, consequently, can perform their pharmacological action locally over time following implantation;
- deactivating the vacuum pump 27 and, in any case, disconnecting the tap 24 from the source of sub-atmospheric pressure;
- completing the filling of the inner volume of the bell 11 (which, in the conditions described above, can occur in a short period of time, this also being typically less than approximately 60 seconds) with the possible closure of the safety element 28 and turning the tap 24 to an emptying position;
- opening the clamp 17, with the consequent release therefrom of the bell 11 which is opened with the consequent possibility of gaining access to the container 3; and
- releasing and opening the container 3 itself by removing the sealing layer 5 and extracting the prosthesis V ready for use.

Tests conducted by the Assignee show that this series of operations can be effected rapidly in surgery in not more than three minutes, including the time necessary to prepare the device 10. In practice, the treatment described above, which leads to the complete removal of bubbles from the prosthesis (and its possible impregnation with drugs), can easily be achieved in a short time interval, thus making it completely compatible with the normal operation times.

The arrangement according to the invention is thus based on the principle of locating the prosthesis to be treated in a treatment chamber which is then taken to a level of sub-atmospheric pressure. This chamber is then connected to a liquid supply line (the bag 23, in the embodiment illustrated). Drawn by the sub-atmospheric pressure in the chamber (and possibly driven by an external over-pressurisation applied using known means, not specifically illustrated in the drawings: for example, squeezing the bag 23, even manually can be sufficient), the liquid completely saturates the apertured parts of the prosthesis, preventing any air bubbles remaining or becoming trapped in the apertures.

As already said, the liquid can be inert (such as, for example, physiological saline) or it can be a vehicle for active principals such as, for example, drugs. In this way, it is possible to obtain the additional result of saturating the apertured parts of the prosthesis with pharmacologically active principals that can be released gradually at the implantation site, effecting a local action over time.

All of this has the further advantage that the nature and quantity of drug can be from time to time selected by the surgeon or surgeons depending on the specific implantation requirements, and this decision can be taken just moments before proceeding to implant the prosthesis.

The practical effectiveness of the arrangement described above has been tested by producing a device that enables the detection of any residual air trapped within the tissue meshes of the suture rings of valve prostheses currently produced by the Applicant. The test device (not specifically illustrated in the drawings) enables a vacuum level to be achieved that corresponds to a pressure of not less than -20 mbar (therefore, a level close to the surface tension of water) on to beakers containing two suture rings immersed in an aqueous solution, the first treated using the method described above and the second comparison ring saturated with water for simple immersion. This is all to extract any air present in the mesh of the two suture rings.

In practice, the test device was formed with a vacuum pump connected to a bell having an observation window. This circuit was subjected to the action of a Jofra LPCA hand-operated vacuum calibrator in order to bring the pressure to a level not less than -20 mbar. Two beakers full of a previously de-gassed aqueous solution were introduced into the bell. The two suture rings (one treated according to the invention, and the other as a comparison) described above were immersed in these beakers.

The behaviour was recorded by video recorder through the observation window in the bell.

Three successive tests were performed. In all three, no escape of residual air was detected in the suture rings treated according to the invention and, therefore, no residual air was present. On the other hand, the escape, and therefore the presence, of air was clearly detected in the untreated rings.

The device 10 shown in Figure 1 corresponds to just one of the many possible embodiments of the invention and, the basic principle of the invention remaining the same, the arrangement described lends itself to many variations, some of which are particularly significant.

For example, even without substantially modifying the structure of the device shown in Figure 1, reference can be made to an arrangement in which the collection bowl indicated above is integrated into the structure of the clamp 17, or an arrangement in which the pillar 24 that supports the bag 23 is integrated with the structure of the clamp 17. This latter can also integrate part of the vacuum bell 11 in its interior.

In complementary manner, the bell 11 can itself be formed so as firmly to seal the vacuum without using an external structure such as the clamp 17: the most immediate example is that of the containers for keeping grocery products sealed, for example, of the type currently known as an albarello seal.

In addition, it is possible to envisage the utilisation of the container in which the prosthesis is packed before installation as the bell for the vacuum 11.

For example, with reference to the arrangement illustrated in Figure 3, one of the half-shells 1 can have a connector such as the connector 16 (the use of the same reference numeral as utilised in Figure 1 is intended to show this possible correspondence) with the possible modification of the mechanism for coupling the two half-shells 1 and 2, for example, using a sealing ring such as an O-ring. This is so as to be able to utilise directly the casing defined by the half-shells 1 and 2 sealingly coupled together as a vacuum bell. To this end, the connector 16 is configured so as to enable the connection to a source of sub-atmospheric pressure (for example, the pump 27 via the line 26). At the same time, the connector 16 is configured as means for introducing into the casing formed from the half-shells 1, 2 the liquid intended to saturate the apertured structure of the prosthesis due to the liquid being drawn into the casing because of the level of sub-atmospheric pressure induced in the casing itself. This therefore means that the casing of the prosthesis container is directly connectable to a source of sub-atmospheric pressure (the pump 27) and a source of liquid (the bag 23) in order to effect the treatment described above without having to prepare a vacuum bell 11 for this purpose.

It goes without saying that, to this end, the container (in this case, the casing defined by the half-shells 1, 2) must have a structure that ensures the integrity of the prosthesis contained therein when the level of sub-atmospheric pressure required therein (for example, -850 mbar) for the treatment described above is present.

Similarly, one or more arcuate slots, indicated 200, are usually present in the lower half-shell 2 of the container shown in Figure 3. These slots are normally closed by one or more sheets, for example, of the material known as Tyvek™ already mentioned above, which is able to act as a sterile, fluid-permeable barrier. Naturally, when the prosthesis container is to be utilised as the vacuum bell, these slots must be closed, for example, with one or more elements (for example, self adhesive) acting as a seal or sealing plug, as schematically indicated 201 in Figure 3.

In particular, where it is integrated with a container, the connector 16 can be a different shape from the luer type of connection referred to specifically in Figure 3. It can, for example, utilise a different kind of luer connection (for example, a male connector in place of a female connector, or vice versa), or a closure element such as a perforable membrane which can be pierced with a needle, the axial lumen of which leads to the tap 24. As is known, a luer connection can be integrated with an arrangement that uses a perforable membrane.

The choice of one of these arrangements, or alternative connection arrangements that are well known to the man skilled in the art, is clearly correlated to the nature of the container utilised as the vacuum bell and/or the requirement of ensuring the necessary conditions of sterility of the connector before use.

An arrangement can also be proposed in which the vacuum bell (whether separate or constituted by the prosthesis container) includes a tank for the filling liquid, thus avoiding the necessity of having to use an external reservoir such as the bag 23 and the connection line (indicated 25 in Figure 1), and the associated elements.

It is equally clear that the reference to a cardiac valve prosthesis provided with a suture ring, as an apertured part, is purely by way of example. The arrangement according to the invention in fact lends itself to be utilised with any prosthesis including at least one part apertured in the terms referred to in the introduction to the present description. It can therefore be any kind of prosthesis (to give an example, a vascular prosthesis, including a prosthesis made from microporous material such as a vascular prostheses made from expanded PTFE) which can be introduced directly into the vacuum chamber 11 or located in this treatment chamber when the prosthesis itself is still in an associated sterile container, such as the inner container 3 of Figure 3, with the requirement of ensuring the accessibility of the apertured part of the prosthesis by the treatment liquid remaining, of course, the same.

It follows therefore that, the principle of the invention remaining the same, the details of construction and the embodiments can be widely varied with respect to that described and illustrated, without by this departing from the ambit of the present invention.

## Claims

1. A method for the treatment of a prosthesis having an apertured structure, characterised in that it includes the stages of:
- providing a holding chamber (11) for the prosthesis (V),
- producing (27) a level of sub-atmospheric pressure in the said holding chamber (11); and
- introducing a liquid (23) into the said chamber (11) which is taken to sub-atmospheric pressure, which liquid saturates the said apertured structure.

2. A method according to Claim 1, characterised in that the level of sub-atmospheric pressure is chosen so that the said liquid substantially completely saturates the said apertured structure so that following the saturation by the said liquid, the said apertured structure is substantially free of air.

3. A method according to Claim 1 or Claim 2, characterised in that the said liquid is a substantially inert liquid.

4. A method according to Claim 3, characterised in that the said liquid is physiological saline.

5. A method according to Claim 1 or Claim 2, characterised in that the said liquid contains a drug.

6. A method according to any of Claims from 1 to 5, characterised in that the said chamber (11) is taken to sub-atmospheric pressure by its connection to a vacuum line (26, 27), and in that the said liquid (23) is made to flow into the chamber (11) at sub-atmospheric pressure by the connection of a liquid supply line (25), with an interruption in the connection of the said vacuum line (26) to the said chamber (11).

7. A method according to any of Claims from 1 to 6, characterised in that the said chamber (11) is taken to a vacuum level of approximately -850 mbar.

8. A method according to Claim 6, characterised in that the said chamber (11) is connected to the said vacuum line (26) for a time interval of less than approximately 60 seconds.

9. A method according to any preceding claim, characterised in that the said liquid is made to flow into the said chamber (11) at sub-atmospheric pressure for a time interval of less than approximately 60 seconds.

10. A device for the treatment of a prosthesis having an apertured structure, characterised in that it includes:
- a casing (11) defining a holding chamber for the said prosthesis; the said casing (11) including opening means (13) for the introduction of the prosthesis into the said chamber (11) and pneumatic sealing means (14),
- a first fluid line (16, 24, 26) leading to the said casing (11) for connecting the said chamber to a source (27) of sub-atmospheric pressure,
- a second fluid line (16, 24, 25) for connecting the said chamber (11) to a source of liquid (23), and
- fluid commutation means (24) acting on the said first (16, 24, 26) and second (16, 24, 25) fluid lines to connect the said chamber (11) in succession with the said source of sub-atmospheric pressure (27) and the said source of liquid (23).

11. A device according to Claim 10, characterised in that it further includes a container (23) which contains the said liquid as the liquid source.

12. A device according to Claim 11, characterised in that the said liquid is a substantially inert liquid.

13. A device according to Claim 12, characterised in that the said liquid is physiological saline.

14. A device according to Claim 11, characterised in that the said liquid contains a drug.

15. A device according to any of Claims from 10 to 14, in the form of a kit in a sterile container.

16. A device according to any of Claims from 10 to 14, characterised in that it further includes a vacuum pump (27) as a source of sub-atmospheric pressure.

17. A device according to any of Claims from 10 to 16, characterised in that a safety cut-off element (28) is interposed in the said second fluid line (16, 24, 25), selectively activatable to open or close the said second fluid line (16, 24, 25).

18. A device according to any of Claims from 10 to 17, characterised in that a filtration element constituting a sterile, fluid-permeable barrier is interposed in the said first fluid line (16, 24, 26).

19. A device according to Claim 10, characterised in that the said fluid commutation means (24) is a tap.

20. A device according to any of the preceding claims from 10 to 19, characterised in that the said casing carries a connector (16) defining a common part of the said first and said second fluid lines.

21. A device according to Claim 20, characterised in that the said connection is a luer connection.

22. A device according to any of Claims from 10 to 21, characterised in that the said casing has two complementary parts (12, 13) connectable together with the interposition of a sealing element (14).

23. A device according to Claim 22, characterised in that locking means (17) for holding the two complementary parts of the casing sealingly connected together are associated with the casing (11).

24. A container for a prosthesis having an apertured structure utilisable for performing the method according to Claim 1, the said container including:
- a casing (1, 2) defining a holding chamber for the prosthesis, and
- at least one duct (16) associated with the said casing (1, 2) and configured to enable the connection of the said prosthesis holding chamber to a source of sub-atmospheric pressure (27); the said casing (1, 2) being sealably closable and having a structure that retains the substantial integrity of the prosthesis in the presence of a level of sub-atmospheric pressure within the said prosthesis holding chamber,
- means (16) for introducing a liquid into the said prosthesis holding chamber held at sub-atmospheric pressure, which liquid saturates the said apertured structure of the prosthesis as a result of the liquid being drawn into the said holding chamber due to the said sub-atmospheric pressure.

25. A container according to Claim 24, characterised in that the said duct and the said liquid introduction means are joined in a single connector (16).

26. A container according to Claim 25, characterised in that the said connector is a luer connector, or of the type that can be pierced with a needle.
